# EUROPEAN PATENT APPLICATION

(11) **EP 4 717 252 A1**
(43) Date of publication of application: **01.04.2026**
(21) Application number: 25175037.8
(22) Date of filing: 08.05.2025
(51) Int. Cl.: A61K 8/06, A61K 8/44, A61K 8/73, A61Q 1/00, A61Q 5/00, A61Q 19/00

(54) **SURFACE TREATED HYDROPHOBIC CELLULOSE**

(30) Priority: 30.09.2024 US 202418902839
(71) Applicant: Miyoshi America Inc., Dayville, Connecticut 06241 (US)
(72) Inventor: TAKEKAWA, Shoji, Shrewsbury, 01545 (US); OHARA, Miwa, Allendale, 07401 (US)
(74) Representative: J A Kemp LLP

(57) **Abstract**

The present disclosure relates generally to cellulose powders having improved dispersibility, feel, and stability in which the powders are surface modified with at least a modified dextrin and/or a modified amino acid to render the cellulose powder hydrophobic. The powders may be used in cosmetic compositions such as a powder foundation, liquid foundation, point makeup, lip, mascara, eyeliner, skin care products which are skin cream, hair care products which are shampoo, conditioner, treatment and hair styling products, hair color products, cleansing products which are a body soap, hand soap and facial cleanser.

## Description

### FIELD OF THE INVENTION

The present disclosure relates generally to cosmetic powder materials formed from cellulose having a surface treated with at least a dextrin or a modified amino acid that imparts hydrophobicity to the cellulose thereby rendering its use in cosmetic formulations feasible. The disclosure relates to the surface treated cellulose, methods of making a cellulose powder surface treated with at least one dextrin and/or a modified amino acid, and cosmetic formulations and uses thereof in which the cosmetic formulations contain the surface treated cellulose. The cosmetic powders have improved stability, and improved emulsion stability.

### BACKGROUND

The information provided below is not admitted to be prior art to the present disclosure, but is provided solely to assist in a more complete understanding of the embodiments.

A significant amount of various powders are conventionally used for making makeup, skincare products, toiletries, and other products marketed and distributed by the personal care industry. Powders dispersed in various product forms such as water base solution, water gel, w/o and o/w emulsion-containing formulations, may suffer from poor dispersibility and product stability, which can result in the formation of aggregates, agglomerates and flocculation. These results can be due to the nature of powder's physical properties, including particle size, surface activity, charge, polarity and specific gravity, to name a few.

Untreated powder agglomerates easily due to several surface properties (including surface charge, surface polarity etc.). In order to solve this problem and to thereby improve dispersibility and stability of powders, surface treatments with various treating agents have been proposed. Agents and methods for surface treating powders vary depending on the aim of the treatment. A treating agent may be selected in view of properties of the surface to be treated and its interaction with a dispersion medium. Known methods include, for instance, lipophilization with oils or metal soaps, hydrophilization treatment with surfactants or silica, and hydrophobization with silicone oils.

There are many types of lipophilization treatments, but there are relatively few known conventional hydrophilization treatments, such as silica treatments. Current hydrophilization treatments that are known are not entirely satisfactory. For example, in a composition formulated with a hydrophilic treated powder, the treating agent sometimes separates from the powder to cause agglomeration of the powder. This may result in mottles and color differences between the desired coating color and the resultant applied color. In addition, re-dispersibility sometimes worsens, which is inconvenient in its use and may cause product stability issues. Further, some of the known surfactants used in aqueous system cause skin irritation, which is problematic in personal products.

In recent years, powders have been developed to provide long lasting cosmetics with a smoother consistency. In obtaining these desirable traits, the focus has largely been on the hydrophobic properties of the surface treatments on powders and pigments, and improvements in the dispersibility of surface treated powders into an oil phase. However, when powders are used in cosmetic systems, such as foundations, lip sticks, lotions, or creams, the powders typically have to be dispersed in an aqueous phase, due to the hydrophilic nature of most cosmetic powders. To disperse non-hydrophobic powders in an aqueous phase, multiple emulsifiers are often used. Without these emulsifiers, dispersions in water-based systems often become problematic. The use of emulsifiers can be disadvantageous, however, with respect to producing a sticky, heavy feeling to the composition.

The affinity of a powder is dependent on surface characteristics of the powder, such as particle size, particularly nano-sized and micro-sized powders and the aspect ratio of powder. The high affinity of residual powders on the substrates that are commonly used in skin and hair consumer products often requires additional wash steps or specialized cleansing products to remove them completely. It also is known that direct contact of inorganic and organic cosmetic powders with the skin may lead to the absorption of water on the skin surface, thus altering the natural hydrophilic and lipophilic balance, which may cause localized dehydration effects and consequently an unpleasant feeling by those using these products. Cosmetic powders therefore are typically treated to modify the surface of the powder to provide improved dispersibility, homogeneity and stability and to reduce the deleterious effects caused by direct contact with the skin.

There are proposed a variety of surface-treating methods. In one method, a silicone oil (for instance, methyl polysiloxane, methyl hydrogen polysiloxane or alkyl silane with the number of carbon atoms of an alkyl portion being not more than 10) is dissolved into a solvent as a surface-treating agent, which then is added and mixed into a powder, and the surface treatment is baked onto the powder by heating after the drying process. In another method, while a powder and octyl triethoxy silane or the like are being dispersed into an organic solvent by using a media grinder, the surface of the powder is treated with an organic silicon compound such as octyl triethoxy silane (JP-A 08-104606). Another method involves stirring and mixing with a Henschel mixer N-octyl trimethoxy silane or N-octyl triethoxy silane as an alkyl silane compound, and a reaction is completed with the powder under heating, and the resultant treated powder is pulverized by a hammer mill (JP-A 2001-181136). In another method, a silicone compound such as methyl hydrogen polysiloxane or the like is emulsified by dispersing it in water, and surfaces of powder particles are coated by mixing the emulsion to the powder (JP-A 09-268271).

JP-B 06-59397 discloses a jet method in which after a metal soap, an organic silicon compound in which a reactive group such as a hydrogen group or the like is bonded to a silicon atom, and a powder are mixed, the mixture is pulverized by a miller using an ejecting stream simultaneously with the surface treatment. JP-A 2002-80748 discloses a method in which in order to improve dispersability of a powder, coating is effected with surface treating agents for an A layer and a layer B by a jet method. Another method involves mixing a silica compound in water, ethanol and aqueous ammonia, and therein dispersing titania powder to prepare a pre-mix 1. Separately, tetraethoxysilane, water and ethanol were mixed to prepare pre-mix 2. Pre-mix 2 was added to pre-mix 1 under stirring with a magnetic stirrer, at a constant rate over 2 hours. The mixture obtained was aged for 12 hours. The coating formation and aging were performed at 25° C. Thereafter, the solution was filtered by suction and the filtrate was dried with hot air at 50° C. for 12 hours to obtain silica-coated powder. This process is disclosed in U.S. Patent No. 6,534,044, the disclosure of which is incorporated by reference herein in its entirety.

U.S. Pat. No. 5,496,544, the disclosure of which is incorporated by reference herein in its entirety, discloses a skin cosmetic composition consisting of an anhydrous powder comprising a solid powder phase mixed with a fat-based binder which contains a silicone mixture comprising at least one silicone oil, at least one silicone wax, at least one silicone resin, and optionally at least on silicone rubber and optionally at least one phenyl dimethicone. However, in U.S. Pat. No. 5,496,544, the anhydrous powder undergoes a physical treatment by said fat-based binder. Therefore, in the cosmetic composition from U.S. Pat. No. 5,496,544, the absence of a covalent chemical bond between the powder phase and fat-based binder has the drawback of an easy extraction of the latter from the powder phase. Also, in the cosmetic composition from U.S. Pat. No. 5,496,544, the powder phase coating consists of complex mixtures of silicones which confer a different kind of sensorial effects on the skin itself.

EP 1 116 753 describes a powder treated with reactive silicone comprising a powder surface-coated with a silicone compound, in which the amount of hydrogen generated from Si-H groups left on the surface of the silicone-treated powder is not greater than 0.2 ml/g of the treated powder and a contact angle between the water and the treated powder is at least 100°. However, the direct reaction between methyl hydrogen polysiloxane containing reactive Si-H bonds and the powder surface described in EP 1 116 753 fails to reach completion and it has the disadvantage to release some H₂ over time, which is the cause of several drawbacks for the obtained cosmetic powder. Indeed, on the one hand the generation of H₂ may cause the containers carrying the powder to swell and deteriorate, on the other hand the powder itself may harden and break.

U.S. Pat. No. 6,482,441, the disclosure of which is incorporated by reference herein in its entirety, discloses powders coated with a layer A and a layer B, where layer A contains organo polysiloxanes, polyolefins, hydrogenated lecithin, N-acylamino acids, and dextrin fatty acid esters, and layer B contains organo polysiloxanes modified at their sole terminal ends with a functional group, alkylsilanes modified at their sole terminal ends with a functional group, and branched fatty acids. The coated powders are said to exhibit super-dispersibility. Surface treatments with N-acylamino acids have been proposed in, for example Japanese Patent Kokai Publications JP-A-61-737775, JP-A-61-69709, JP-A-3-200879, JP-A-5-186706, JP-A-9-328413, and JP-A-10-226626, whilst a method for coating with fatty acids is proposed in Japanese Patent Kokai Publications JP-A-60-69011 and the like, and a method for surface-treating with hydrogenated lecithin is proposed in Japanese Patent Kokai Publications JP-A-60-184571, and JP-A-60-190705.

Cellulose is a plant-based product, and therefore is an environmentally friendly material, as opposed to most cosmetic powders that are made from polymeric materials, inorganic materials, and the like, which do not biodegrade. Cellulose has, however, strong hydrophilicity and lower reactivity than most cosmetic powders, and applying a hydrophobic surface treatment to the cellulose has proven difficult.

It would be desirable to provide environmentally conscious cosmetic powders that are suitable for use in cosmetic compositions in which the powders have been surface treated to improve the powder's dispersibility and stability in the composition. Other features and advantages will become apparent from the following disclosure. While certain drawbacks and disadvantages have been described with respect to the state of the art, the embodiments described herein are not to be construed to exclude some or all of the features described above. Indeed, aspects of the embodiments may include features known in the art, without suffering from their previously known adverse effects.

### SUMMARY OF INVENTION

The embodiments described herein relate to a surface treated cellulose powder in which the powder has been surface treated with at least a modified dextrin and/or a modified amino acid, and salt forms thereof. In accordance with an embodiment, there is provided at least one cosmetic powder in which the surface of the at least one cosmetic powder is chemically modified with at least one polysaccharide or mixtures thereof, and salt forms thereof, wherein the polysaccharide is chemically immobilized on the surface of the at least one powder.

Another embodiment relates to a method for making a surface treated cellulose powder that includes: (a) preparing a mixture of modified dextrin and/or a modified amino acid and at least one cellulose powder; (b) optionally heating the mixture; (c) agitating the mixture to uniformly disperse the cellulose powder and modified dextrin and/or a modified amino acid to form a surface treated cellulose mixture; (d) heating the surface treated cellulose mixture during or after agitating in (c); and (e) drying the surface treated cellulose mixture to remove water and optional solvent to form a surface treated cellulose powder.

According to other embodiments, there is provided a cosmetic formulation that includes: (a) at least one surface treated cellulose powder in which the surface of at the least one cellulose powder is treated with modified dextrin and/or a modified amino acid; and (b) a cosmetically acceptable carrier.

Still other aspects and advantages of the embodiments will become readily apparent to those having ordinary skill in the art from the following detailed description, wherein particularly preferred embodiments are shown and described, simply by way of illustration. As will be realized the preferred embodiments include other and different embodiments, and its several details are capable of modifications in various obvious respects. Accordingly, the description is to be regarded as illustrative in nature and not as restrictive.

### BRIEF DESCRIPTION OF DRAWINGS

The embodiments can be understood from the following detailed description when read in connection with the accompanying drawings. Included in the drawings are the following figures:
Figure 1 includes photographs of samples of cellulose powder treated with various surface treatment agents A-G in aqueous solution, where surface treatments A and B exhibit superior hydrophobicity than C-G.
Figure 2 includes photographs of emulsions prepared with cellulose powder treated with surface treatment agent A, at 250X magnification, and at various processing conditions, showing emulsion stability.
Figure 3 includes photographs of emulsions prepared with cellulose powder treated with surface treatment agent B, at 250X magnification, and at various processing conditions, showing emulsion stability.

It is to be noted, however, that the appended drawings illustrate only typical embodiments of this invention and are therefore not to be considered limiting in its scope, for the invention may admit to other equally effective embodiments.

### DETAILED DESCRIPTION OF A PREFERRED EMBODIMENT

Reference is made to the figures to illustrate selected embodiments and preferred modes of carrying out the invention. It is to be understood that the embodiments are not hereby limited to those aspects depicted in the figures.

The following definitions and non-limiting guidelines are provided to assist in better understanding the detailed description of herein. The headings (such as "Background" and "Brief Summary,") and sub-headings used herein are intended only for general organization of topics within the disclosure of the embodiments, and are not intended to be limiting. For example, subject matter disclosed in the "Background" may include aspects of technology within the scope of the embodiments, and may not constitute a recitation of prior art. Subject matter disclosed in the "Brief Summary" is not an exhaustive or complete disclosure of the entire scope of the embodiments. Classification or discussion of a material within a section of the specification as having a particular utility (e.g., as being an "active" or a "carrier" ingredient) is made for convenience, and no inference should be drawn that the material must necessarily or solely function in accordance with its classification herein when it is used in any given composition.

The citation of references herein does not constitute an admission that those references are prior art or have any relevance to the patentability of the embodiments disclosed herein. Any discussion of the content of references cited in the Background is intended merely to provide a general summary of assertions made by the authors of the references, and does not constitute an admission as to the accuracy of the content of such references.

The description and specific examples, while indicating embodiments, are intended for purposes of illustration only and are not intended to be limiting. Moreover, recitation of multiple embodiments having stated features is not intended to exclude other embodiments having additional features, or other embodiments incorporating different combinations the stated of features. Examples are provided for illustrative purposes of how to make and use the compositions and methods described herein, unless explicitly stated otherwise, are not intended to be a representation that given embodiments have, or have not, been made or tested.

As used herein, the words "preferred" and "preferably" refer to embodiments that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope thereof. In addition, the compositions and the methods may comprise, consist essentially of, or consist of the elements described therein.

As used throughout, ranges are used as a short-hand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls.

Throughout this description, the use of the term "about" or "approximately" is intended to denote an approximation of the number, which includes the number modified by the term, and a reasonable deviation from that term, including standard measurement errors. Unless otherwise specified, all percentages and amounts expressed herein and elsewhere in the specification should be understood to refer to percentages by weight. The amounts provided are based on the active weight of the material. The recitation of a specific value herein is intended to denote that value, plus or minus a degree of variability to account for errors in measurements. For example, an amount of 10% or about 10% may include 9.5% or 10.5%, given the degree of error in measurement that will be appreciated and understood by those having ordinary skill in the art.

As used herein, the term "cosmetic composition" means a composition that is intended to be applied onto the consumer's skin, particularly onto the facial skin or onto the body skin area or onto hair, so as to regulate the condition of the skin and/or to improve the appearance of the skin and hair. The term "powder" denotes any material having a particle size within the range of from about 0.01 micrometer to 1000 micrometers used for cosmetics and other purposes. The term "average primary particle size" of powder treated with polysaccharide denotes the equivalent volume mean primary particle size of the elementary powder treated with polysaccharide. The average primary particle size is measured on the powder treated with polysaccharide, before being treated.

All percentages, ratios and proportions herein are by weight, unless otherwise specified. All such weights as they pertain to listed ingredients are based on the active level, unless otherwise specified.

Embodiments described herein include one or more cellulose powders having been treated with at least one dextrin and/or modified amino acid in which the surface modification imparts sufficient hydrophobicity to the powder to enable its use in a number of industries. Cellulose powder includes cellulose, (C₁₂H₂₀O₁₀)n, having the following formula: 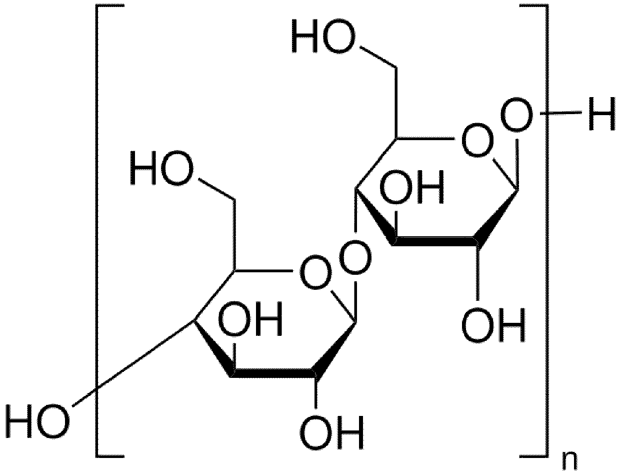 where n is the number of repeating units that typically depends on origin of the cellulosic material, and represents its degree of polymerization (DP). Cellulose is a linear glucose homopolymer that includes glucopyranose units linked by β-1,4-glycoside bonds. Cellulose powder typically is made by mechanical disintergration of α-cellulose usually obtained in pulp form from plant material.

Cellulose power is hydrophilic but insoluble in water. Cellulose powders find many uses, including in pharmaceuticals due to its excellent compressibility (use in tablets and other solid dosage forms), processed food products, cosmetics, as an anti-caking agent, a stabilizer, a texture modifier, or a suspending agent. Cellulose powder has characteristics such as thickening, emulsification stability, water retention, oil absorption, shape retention, etc., so food additives, tablet excipients, dispersants, shape retention agents, water retention agents, filter aids It is used in a wide range of fields such as foods, medicines, cosmetics, building materials, ceramics, rubber, plastics, etc. as fillers, additives for paints and adhesives.

Cellulose powder can range in particle size from 10 to 500 µm, or from about 25 to about 400, or from about 30 to about 300, or from about 50 to 250 µm, or any and all values therebetween. Cellulose powders are commercially available from a variety of sources, with a variety of average particle sizes ranging from about 50 µm, 75 µm, 100 µm, and 250 µm. In some embodiments, cellulose powders useful herein may have: an average degree of polymerization of 100 to 1,000; a weight average particle size of over 30 µm, but less than 250 µm; an apparent specific volume of 2 to less than 15 cm³/g; and an organic carbon content from residual impurities, which is defined by the total organic carbon content (%) during 1% NaOH extraction to the total organic carbon content (%) during pure water extraction, of over 0.07 to 0.3%.

Cellulose is hydrophilic, but for some applications, it may be desirable to apply a hydrophobic surface treatment to the cellulose powder materials. The present inventor unexpectedly discovered that a number of agents known to be useful in applying hydrophobic surface treatments, were not useful with cellulose powder materials. For example, fatty acid esters (e.g., hydrogenated Olive Oil Stearyl Esters), silicone-based materials (e.g., dimethicone), disodium stearoyl glutamate, lecithin, fatty acids such as isostearic acids, and the like were not useful in effective in rendering hydrophobic the surface of the cellulose powder materials. The inventor discovered, quite unexpectedly, that a modified dextrin compound, and/or a modified amino acid (other than disodium stearoyl glutamate, which is excluded herein), were able to effectively impart hydrophobicity to the surface of cellulose powder materials, and render the modified cellulose powders useful in forming stable emulsions.

A modified dextrin may be used in some embodiments as a surface treatment agent for cellulose powder materials. Dextrins are low molecular weight carbohydrates that include mixtures of D-glucose units linked by glycosidic bonds. Dextrins include a variety of products produced by heating a starch in the presence of a small amount of moisture and acid. Dextrins may include yellow or canary dextrins, white dextrins, British gums, amylodextrin, erythrodextrin, and Achrodextrin. typically has the molecular formula (C₆H₁₀O₅)n, where n is an integer representing the number of glucose molecules in the chain, which provides the length and molecular weight of the particular dextrin. Typically, n ranges from 2-15, or from 3-9. Dextrin has the following chemical formula:

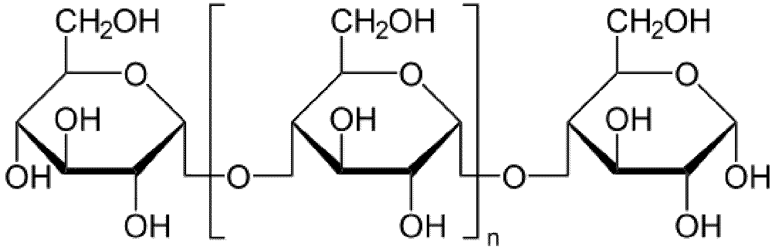

Any modified dextrin can be used in the embodiments so long as the modified dextrin is capable of treating the surface of a cellulose powder material to improve its hydrophobicity. In an embodiment, the modified dextrin is a dextrin ester, and specifically, the modification includes modification by at least one preferably C₈-C₂₄ and in particular C₈-C₂₀ fatty acid ester of dextrin, and more particularly C₁₂, C₁₄, or C₁₆ fatty acid esters of dextrin. The dextrin ester may be selected from dextrin myristate and/or dextrin palmitate, and mixtures thereof. Dextrin palmitate may be available, for example, from those sold under the names Rheopearl TL^{®} and Rheopearl KL^{®} by the company Chiba Flour.

In addition to, or as an alternative to the modified dextrin, the cellulose powder also may be surface treated with a modified amino acid to impart hydrophobicity to the powder materials. In an embodiment, the modified amino acid is an acylamino acid in which a fatty acid is conjugated, or amidated to an amino acid. Suitable amino acids or amino acid salts are any known α-amino acids that can be acylated with fatty acid halides to form N-acylamino acids. Suitable amino acids include, for example, glutamic acid, sarcosine, aspartic acid, alanine, valine, leucine, isoleucine, lysine, proline, hydroxyproline, glycine, serine, cysteine, cystine, threonine, histidine and salts thereof and, more particularly, glutamic acid, sarcosine, aspartic acid, glycine, lysine and salts thereof. Glutamic acid, aspartic acid, glycine, and lysine are particularly preferred. The amino acids may be used in optically pure form or as racemic mixtures.

The acylamino acid can be modified with any fatty acid, including modification by at least one preferably C₈-C₂₄ and in particular C₈-C₂₀ fatty acid, and more particularly C₁₂, C₁₄, or C₁₆ fatty acid. In one embodiment, the modified amino acid is a stearoyl amino acid, or stearoyl glutamic acid, stearoyl aspartic acid, stearoyl lysine, stearoyl glycine, and mixtures thereof. In another embodiment, the modified amino acid is stearoyl glutamic acid.

In order to facilitate or enhance immobilization of surface-treatment agents to the cellulose powder material, a reaction may be created by a water soluble compound having a lipophilic or hydrophilic moiety being absorbed onto the surface of the cellulose powder. As a non-limiting example, addition of a water-soluble salt of a polyvalent metal, such as magnesium, calcium, aluminum, titanium, zinc or a zirconium salt (e.g., zirconium sulfate or chloride), or an alkaline salt, such as a sodium, potassium, lithium, ammonium, or an amine salt, can produce a chemical linkage. These metals typically are present in the form of a salt, such as a sulfate salt (e.g., aluminum sulfate, and the like). The reaction provides a surface-treatment agent chemically immobilized onto the surface of the cellulose powder particle. In contrast, conventional coating a substrate with a surface-treatment agent involves absorbing the surface-treatment agent onto the surface of the substrate or pigment.

During treatment with one or more surface treatment agents, the surface of one or more cellulose powders becomes modified. Including a cosmetically acceptable oil (a single oil or mixture of oils) during a treatment in which the cellulose powder surface is modified invites oil at the same time as the particles become attached or linked to each other. Use of oil is beneficial in the embodiments because modified dextrins and modified amino acids useful in the embodiments are not readily dissolvable in water. In addition, the modified amino acid may be heated to soften the modified amino acid during mixing to enable it to spread out onto the surface of the cellulose powder. Surface treatment agents and oil in combination function as a "glue" to attach or link particles, and other components optionally present, to each other. A mixture of two or more different cellulose powders during such surface treatment results in forming composites, which are typically randomly and uniformly distributed onto the surface. Thus, oils, emulsifiers, etc., can be present in a mixture with one or more cellulose powders when contacted with a surface treatment agent.

Following surface modification, a cellulose powder material can then be admixed or blended with another (e.g., second) powder material, such as a pigment, substrate, or extender, or another cosmetically acceptable ingredient such as an oil, emulsifier, binder, etc. The additional material(s) may or may not have been treated with a surface treatment agent. Alternatively, two or more materials (e.g., different colored pigments together with the cellulose powder material), can be combined or mixed together prior to contact with a surface treatment agent, such as in an aqueous slurry, and then subsequently contacted with a modified dextrin and/or modified amino acid in order to simultaneously produce two or more surface modified or treated materials. Chemical immobilization of the modified dextrin and/or modified amino acid, and optionally other surface treatment agents on the powder materials can be facilitated by a water soluble compound having a lipophilic or hydrophilic moiety being absorbed onto the surface of the material, as set forth herein or known to the skilled artisan.

Additional cosmetic powders and pigments can be used together with the modified cellulose powders. These include, for example, substrates including clay, mica (e.g., pearl colored mica, such as Timron Super Silver^{™}, a mica coated with titanium dioxide produced by Rona/EMD Industries), talc, kaolin, sericite, silica (e.g., silica beads such as aluminum silicate, magnesium silicate and calcium sodium silicate, Beadyl Beads^{™}, fumed silica), alumino-silicate minerals (zeolites), nylon (e.g., nylon beads or nylon powder), acrylates such as polymethyl methacrylate (PMMA or powder), metal powders (such as aluminum), ceramic powders (such as silicon nitride or boron nitride), cotton powder, wool powder, urethane, polystyrene and polystyrene powder, polyolefin, polyethylene and polyethylene powder, polyamide, zirconium, aluminum oxide, zirconium oxide, starch, starch powder and starch derivatives such as aluminum starch octenylsuccinate, and calcium carbonate (chalk).

Substrates also may include "extenders." An extender can function as a filler or bulking agent for powders and dispersions as set forth herein or known to the skilled artisan (e.g., pressed foundation, loose powder, blush, concealer, etc.). Extenders as a class typically have a size, shape or structure that is similar or identical to substrates as disclosed herein and understood by the skilled artisan. The term extender is typically used to refer to a substrate material that is added to a powder or dispersion after surface treatment or modification of cosmetic powder material.

Extenders include natural and synthetic substrates that may or may not have a color, shade, hue, chroma (saturation) or lightness that may vary in saturation and luminance. As with a substrate, an extender has a size typically greater than 1 micron (1 µm), for example about 1-30 microns, and can have various shapes, for example, spherical, elliptical or "platy."

Non-limiting examples of extenders include talc, kaolin (clay), natural and synthetic micas including muscovite mica and sericite, titanated mica, cotton powder, starch, magnesium carbonate, calcium carbonate, aluminum silicate, magnesium silicate, calcium silicate, synthetic silicates, clay, bentonite, montmorillionite, calcite, chalk, bismuth oxychloride, boron nitride, fumed silica, silica beads, plastic beads such as acrylics, nylons such as Nylon 12, nylon beads, aluminum, calcium, or sodium silicate, and barium sulfate.

Additional cosmetic powder materials also may be comprised of pigments. As used herein, the term "pigment," which includes "dyes" is a natural or synthetic material that has a certain color, shade, hue, chroma (saturation) or lightness. Pigments may be organic or inorganic in chemical nature. Pigments typically have a primary particle diameter not greater than about 3 microns. Pigments more typically are about one order of magnitude smaller in size than substrates, for example, about 0.01-1.0 microns in diameter. Other pigments, such as pearl pigments typically have a larger size, for example 10, 20, 30, 40, or 50-100 microns (µm). Thus, the cosmetic powder material, albeit a substrate, a pigment, or other powder, usually has an average particle size within the range of from about 0.01 to about 100 µm, or from about 0.05 to about 50 µm, or from about 0.1 to about 35 µm.

Non-limiting examples of inorganic pigments include white titanium dioxide pigments (e.g., rutile, anatase, and ultrafine TiO₂), zinc oxides (e.g., ultrafine ZnO), which can be of pigment grade and have a primary size of about 0.25 µm, or ultrafine grade, and have a primary size of less than about 0.1µm. Other inorganic pigments include zirconium oxide, zirconium dioxides, iron oxides (including yellow, red, brown, green and black iron oxides), ultramarines (such as ultramarine blue, ultramarine violet, ultramarine pink, etc.), pearl pigments (e.g., mica, titanated mica, bismuth oxychloride, etc.), manganese violet, Prussian blue, chromium oxides, chromium hydroxides, and carbon black. Non-limiting examples of organic pigments include "lake" dyes, β-carotene, carmine, chlorophyll and the like.

The powder material may be an inorganic powder, such as an extender pigment, non-limiting examples of extender pigments include: mica, sericite, talc, kaolin, synthetic mica, muscovite, phlogopite, epidolite, biotite, calcium carbonate, magnesium carbonate, calcium phosphate, alumina, magnesium oxide, aluminum hydroxide, barium sulfate, magnesium sulfate, silicic acid, silicic anhydride, magnesium silicate, aluminum silicate, aluminum magnesium silicate, calcium silicate, barium silicate, strontium silicate, silicon carbide, magnesium aluminate, magnesium metasilicate aluminate, chlorohydroxyaluminum, clay, bentonite, zeolite, smectite, hydroxyapatite, ceramic powder, boron nitride and silica.

The powder material may be a special composite extender pigment such as, but not limited to Excel Mica, Excel Pearl and Powder La Vie sold by Miyoshi Kasei, Inc.; white pigments such as titanium dioxide, zinc oxide and cerium oxide; color pigments such as red iron oxide, yellow iron oxide, black iron oxide, chromium oxide, chromium hydroxide, Prussian blue, ultramarine, inorganic blue pigment, carbon black, titanium oxide, mango violet, cobalt violet, laked tar dye and laked natural dye; bright pigments such as bismuth oxychloride, mica titanium, fish scale guanine, a powder obtained by coating synthetic mica with titanium dioxide, a powder obtained by coating silica flakes with titanium dioxide as sold under a trade name "Metashine" by Nippon Sheet Glass Co., Ltd., a powder obtained by coating alumina flakes with tin oxide and titanium dioxide, a powder obtained by coating aluminum flakes with titanium dioxide, a powder obtained by coating copper flakes with silica as sold by Eckart, U.S.A., a powder obtained by coating bronze flakes with silica and a powder obtained by coating aluminum flakes with silica;

Suitable powder materials include inorganic pigments such as, but not limited to titanium dioxides, zinc oxides, zirconium dioxides, iron oxides (including yellow, red, and black), ultramarines (such as ultramarine blue, ultramarine violet, etc.), and manganese violet. The powder material may be a mixture of any or all of the suitable powder materials.

The cellulose powder whose surface has been modified with at least one modified dextrin and/or modified amino acid usually will have an average treatment ratio of from about 0.1 to about 15%, by weight of modified dextrin and/or modified amino acid (including salt forms) by weight per 100 parts by weight of cellulose powder. The cellulose powder also may be treated with from about 0.1 to about 10%, by weight of modified dextrin and/or modified amino acid, or from about 1 to about 8% powder, by weight of modified dextrin and/or modified amino acid, or from about 1 to about 7% powder, by weight of modified dextrin and/or modified amino acid. The amount of modified dextrin and/or modified amino acid surface treatment agent also may vary depending on the type of cellulose powder. For example, for ultrafine cellulose powder, more modified dextrin and/or modified amino acid may be used, e.g., double or triple the amount used for powder having smaller surface area. Using the guidelines provided herein, persons having ordinary skill in the art will be capable of determining an appropriate amount of modified dextrin and/or modified amino acid surface treatment agent to use, depending on the type of powder being treated.

The surface treated cellulose powder of the embodiments therefore can be comprised of at least one cellulose powder having a modified surface in which at least one modified dextrin and/or modified amino acid, or salts thereof, is chemically immobilized on the surface of the powder material. The treated powder material may be used as is in a cosmetic composition, or it may be further treated with one or more additional surface treatment agents. Other cosmetic powders may be mixed together with the cellulose treated powder (before or after surface treatment) and formulated into a suitable composition. These additional cosmetic powders may be treated with one or more additional surface treatment agents. Specific non-limiting classes of surface treatment agents include surface active agents, which include surfactants, detergents, wetting agents and emulsifiers. Surface-active agents may be nonionic, anionic, cationic, amphoterics, hydrophobic or hydrophilic.

Surface-treatment agents for cosmetic powders other than the cellulose powders discussed herein typically have one or more reactive groups, such as a hydrophilic moiety (e.g., a carboxyl group, a phosphorous group, a sulfur group, a silanol group or a silane group) or hydrophobic moiety (e.g., a hydrocarbon, a dialkyl(CH₃-, C₂H₅-) polysiloxane, perfluoroalkyl, etc.) in their structure. Surface-treatment agents may or may not contain one or more hydroxyl groups or alkylene oxide moieties, such as ethylene oxide or propylene oxide. Those having hydroxy groups in their structure and hydrophilic characteristics can be delivered after completing the reaction onto the surface.

Non-limiting examples of surface treatment agents for cosmetic powders other than the cellulose powders discussed herein include acyl collagens, ether carboxylic acids, lactic acid, gluconic acid, galacturonic acid, glucarolactone, gallic acid, glucoheptanoic acid, amino acids (such as thereonine and serine) and their salts, acyl amino acids (such as acylglutamates, acylsarcosinates, acylglycinates, and acylalaninates), fatty acids and their salts, and glycerol phosphate esters (such as lecithin). Additional non-limiting examples of surface-treatment agents include methicone, dimethicone and polyethylenes with free carboxylic acids.

Examples of anionic surface active agents (surfactants) for cosmetic powders other than the cellulose powders discussed herein include soaps (fatty acids/ alkyl carboxylic acids salt), hydroxy fatty acids, alkyl sulfate, alkyl ether phosphate, polyoxyalkylene alkyl ether sulfate, polyoxyalkylene alkyl ether carboxylate, alkylether phosphate, acyl N-methyl taurate, N-acylamino acid salts (glutamate, sarcosinate, lalaninate, glycinate, B-alaninate), acyl peptides (acyl collagen, acyl silk protein), sodium cocoate, stearic acid, iso-stearic acid, potassium palmitate, sodium laurate, 12-hydroxystearic acid, sodium lauryl sulfate, sodium myristyl phosphate, sodium myristoyl sarcosinate, sodium polyoxyethylene lauryl sulfate, polyoxyethylene myristyl carboxylate, potassium myristate, zinc gluconate, isostearyl sebacic acid, sodium myristoyl taurate, disodium stearoyl glutamate, disodium cocoyl glutamate, arginine lauryl glycinate, sodium dilauramidoglutamide lysine.

Suitable surface treatment agents for cosmetic powders other than the cellulose powders discussed herein may include one or more of the surface treatment agents disclosed in, for example, U.S. Patent Nos., 6,887,494, U.S. Patent Application Publication Nos. 2008/0299158, 2011/0318286, the disclosures of which are incorporated by reference herein in their entireties.

The cellulose powders may be used in cosmetic compositions that comprise the surface treated cellulose powder and a cosmetically acceptable vehicle. In an embodiment, the surface treated cellulose powder is present in an amount within the range of from about 0.1% to about 50% by weight of the composition, or from about 0.5% to about 30%, or from about 1% to about 20% by weight, based on the weight of the composition.

Depending on the formulation (e.g., liquid formulation, powder formulation, skin lotion, body soap, Shampoo, Conditioner, Hair Styling, etc.), the amount of the cellulose powder can vary widely. For example, for a powder formulation, such as makeup foundation or the like, the amount of the treated cellulose powder can be used in an amount of from about 5 to about 50% by weight, or from about 15 to about 40% by weight, or from about 25 to about 35% by weight, or at about 30% by weight. For a skin lotion formulation, the amount of the treated cellulose powder can be used in an amount of from about 0.1 to about 15% by weight, or from about 1 to about 10% by weight, or from about 2 to about 7% by weight, or at about 5% by weight. For a body soap formulation, the amount of the treated cellulose powder can be used in an amount of from about 2 to about 40% by weight, or from about 5 to about 20% by weight, or from about 7 to about 15% by weight, or at about 10% by weight

The cosmetic compositions useful in the embodiments described herein also may contain other conventional components useful in various cosmetic compositions. Any cosmetically acceptable vehicle may be used together with the treated cellulose powder material. Such vehicles may include, for example, water, glycerin, dimethicone, beeswax, glyceryl stearate, and the like. Other ingredients normally used in cosmetics also may be present, when desired. For example, inorganic powders such as talc, kaolin, sericite, muscovite, phlogopite, red mica, biotite, synthetic mica, lithia mica, vermiculite, magnesium carbonate, calcium carbonate, diatomite, magnesium silicate, calcium silicate, aluminum silicate, barium silicate, barium sulfate, strontium silicate, wolframic acid metal salt, or silica, hydroxyapatite, zeolite, boron nitride, ceramic powder, organic powders such as nylon powder, polyethylene powder, polystyrene powder, benzoguanamine powder, polyfluoridation ethylene powder, di-styrene benzene polymer powder, epoxy powder, acrylic powder, silicone powder, microcrystalline cellulose, inorganic white pigments such as titanium dioxide and zinc oxide, inorganic red system pigments such as iron oxide (red iron oxide) and titanic acid irons, inorganic brown system pigments such as γ-iron oxides, inorganic yellow system pigments such as yellow soil and yellow iron oxides, inorganic black color system pigments such as tetravalent acid iron oxide, carbon black, inorganic violet system pigments such as mango violet, cobalt violet, inorganic green system pigments such as chromium oxide, chromium hydroxide, and titanic acid cobalt, inorganic blue system pigments such as ultramarine blue, and prussian blue, pearl pigments such as titanium dioxide covered mica, titanium dioxide covered bismuth oxychloride, bismuth oxychloride, titanium dioxide covered talc, fish scale foil, colored titanium dioxide covered mica, metal powder pigment such as aluminum powder, copper powder, colored composite pigments such as iron-doped zinc oxide and iron-doped titanium dioxide.

Other pigments may be used, such as red No.201, red No.202, red No.204, red No.205, red No.220, red No.226, red No.228, red No.405, orange-colored No.203, orange-colored No.204, yellow No.205, yellow No.401 and blue No.404, organic chlorophyll pigment such as FD&C Red No.3, red No.104, red No.106, red No.227, red No.230, red No.401, red No.505, orange-colored No.205, FD&C Yellow No.4, yellow No.5, yellow No.202, yellow No.203, orange-colored No.3 and zirconium, barium, or aluminum lake of blue No.1, natural colorants such as β-carotene, hydrocarbon oils such as squalane, mineral oil, petroleum jelly, micro crystalline wax, ozokerite, ceresin, myristic acid, palmitic acid, stearic acid, oleic acid, iso-stearic acid, cetyl alcohol, hexadecyl alcohol, oleyl alcohol, cetyl 2-ethylhexanoate, 2-ethylhexyl palmitate, 2-octyldodecyl myristate, neo-pentylglycol di-2-ethylhexanoate, glyceryl tri-2-ethylhexanoate, 2-octyldocyl oleate, isopropyl myristate, glyceryl triisostearate, caprylic/capric triglyceride, olive oil, avocado oil, yellow bees wax, myristyl myristate, mink oil, lanolin oil, silicone oil, higher fatty acid oil, ester oils of fatty acids, higher alcohol, oil components of wax groups, cyclopentasiloxanes, dimethicones, trimethylsiloxysilicates, and organic solvents such as acetone, toluene, butyl acetate, and ester acetate can be used in various amounts.

Resins such as alkyd resin, urea-formaldehyde resin, Nylon-12, plasticizers such as camphor, acetyl tributyl citric acid, ultraviolet absorbing agents, antioxidants, antiseptics, emulsifiers, surfactants, stabilizers, defoamers, moisturizing agents, perfumes, water, alcohol, and thickeners can also be used. Non-limiting examples of emulsifiers include cetyl dimethicone copolyol, polygyceryl-4 isosteatrate, glyceryl stearate, PEG-100 stearate, cetyl alcohol, dicetyl phosphate, and ceteth-10 phosphate isostearic acid.

Surfactants typically include nonionic forms. Non-limiting examples of nonionic surfactants include polyoxyalkylene (PEG or/and PPG) type nonionic emulsifiers having structures: 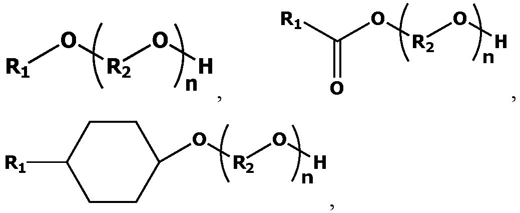 and wherein R₁is selected from the group consisting of alkyl, alkylamide, alkenyl, alkynyl, alkoxy, aryl, cycloalkyl, and arylalkyl group, each of which may be substituted by one or more hydroxy group, and may further be substituted by one or more alkoxyl, carboxyl, or oxo group. R₁ has a carbon number of C₈ to about C₂₄; R₂ is selected from the group consisting of -C₂H₄-, -C₃H₆-, and -C₄H₈-.

The cellulose powder surface treated with at least one modified dextrin and/or modified amino acid can be prepared by preparing a mixture of oil, at least one modified dextrin or a salt thereof, and/or at least one modified amino acid or a salt thereof, and at least one cellulose powder, and optionally heating the mixture. If at least one modified amino acid is used as the surface treatment material, the mixture preferably is heating during the initial formation. The mixture can be agitated with high speed to homogenize the mixture to a homogenized powder mixture that is uniformly dispersed. The homogenized powder mixture then can be contacted with a neutralizing agent (e.g., Al₂(SO₄)₃), to neutralize the homogenized powder mixture, and to chemically immobilize the at least one modified dextrin and/or modified amino acid to the surface of the cellulose powder. The method may further include heating during or after agitation, filtering and drying the surface modified cellulose powder.

In certain embodiments, anywhere from about 1 to about 10 g, or from about 2 to about 8, or from about 3 to about 5 g of modified dextrin and/or modified amino acid, or a salt thereof, is added to from about 90 to 99, or from about 92 to about 98, or from about 95 to about 97 g of an agent suitable to dissolve or disperse the modified dextrin and/or modified amino acid. Preferably, the agent is an oil. The embodiments therefore include the use of anywhere from about 1 to 10 parts by weight of modified dextrin and/or modified amino acid, or salts thereof, or from about 2 to about 8, or from about 3 to about 5, or about 3 parts by weight modified dextrin and/or modified amino acid, or salts thereof, added to from about 90 to 99, or from about 92 to about 98, or from about 95 to about 97 parts by weight of an agent suitable to dissolve or disperse the modified dextrin and/or modified amino acid. The modified dextrin and/or modified amino acid, or salts thereof, can be added to the agent suitable to dissolve or disperse the modified dextrin and/or modified amino acid, at a temperature of from about 25 to about 95 °C, or from about 40 to about 80°C, or at about 70°C, and mixed in a disperser for a period of time sufficient to homogenize the mixture. Any dispersing and/or mixing apparatus can be used. A suitable disperser may include a ROBOMIX^{®} disperser, commercially available from Primix Corporation, Osaka, Japan. The mixture can be mixed for anywhere from about 10 minutes to an hour, or from about 15 minutes to 45 minutes, or from about 18 minutes to 30 minutes, or for about 20 minutes, until the mixture is adequately homogenized.

The cellulose powder then can be added to the homogenized mixture, with stirring. The amount of cellulose powder added will vary, depending on the amount of agent suitable to dissolve or disperse the modified dextrin and/or modified amino acid that is used, and generally is added in an amount of from about 50 to about 150% by weight, based on the weight of the agent, or from about 75% to about 125%, or from about 90% to about 110%, or from about 98% to about 105%, or about the same amount of agent. In one embodiment, from about 50 to about 150 grams of cellulose powder are added, or from about 75 to about 125g, or from about 90 to about 110g, or from about 98 to about 105g, or about 97 g. of powder are added. The cellulose powder can be mixed in the dispersing and/or mixing apparatus for a period of time sufficient to adequately disperse the powder. The powder can be mixed for a period of time, and at a sufficient temperature, until adequately dispersed.

Mixing can take place for a period of time of about 10 seconds to about 5 minutes, or from about 20 seconds to about 3 minutes, of for less than one minute, or for about 40 seconds, and repeated anywhere from 2 to 10 times, or from 2 to 8 times, or about 3 times. In one embodiment, the mixing is carried out for 40 seconds, and repeated 3 times. The temperature in which the components are mixed can range from about about 25 to about 95 °C, or from about 40 to about 80°C, or at about 70°C.

A suitable neutralizing agent may be added to the powder-containing mixture to bring the pH of the mixture to a value within the range of from about 2 to about 10, or from about 3 to about 8, or from about 4 to about 7, or about 4.0. Any neutralizing agent can be used in the embodiments, and a suitable neutralizing agent is aluminum sulfate. The neutralizing agent can be metered into the mixture until the pH reaches the desired value. Once the final pH is reached, the product then can be recovered from the mixture using any suitable mechanism, including filtration, and then drying. The cellulose powder treated with modified dextrin and/or modified amino acid can be dried at a temperature of between about 75 to about 200 °C, or from about 90 to about 150°C, or at about 105°C, for a period of time sufficient to dry the powder. The cellulose powder treated with modified dextrin and/or modified amino acid may be subjected to drying for a period of from about 1 to about 15 hours, or from about 2 to about 10 hours, or from about 4 to about 8 hours, or about 6 hours, to produce, in the embodiments disclosed above, about 100 g of cellulose powder treated with modified dextrin and/or modified amino acid.

The cellulose powder treated with modified dextrin and/or modified amino acid can be used in a cosmetic composition that contains conventional cosmetic additives. For example, the composition may include up to about 25 wt% of a non-volatile oil. The non-volatile oil may be comprised of an organic, UV-active material that functions as a UV-protective agent (a "sun block"). Preferably, two or more organic, UV-actives are used to provide a wide spectrum of protection in the UV region. For example, a combination of at least one UV protecting agent that mainly provides protection against UVA light, and at least one UV protecting agent that mainly provides protection against UVB light, may be used.

A wide variety of conventional UV protecting agents are suitable for use herein. Non-limiting exemplary organic, UV-actives include: 2-ethylhexyl-p-methoxycinnamate (commercially available as PARSOL MCX), butylmethoxydibenzoyl-methane, 2-hydroxy-4-methoxybenzo-phenone, 2-phenylbenzimidazole-5-sulfonic acid, octyldimethyl-p-aminobenzoic acid, octocrylene, 2-ethylhexyl N,N-dimethyl-p-aminobenzoate, p-aminobenzoic acid, 2-phenylbenzimidazole-5-sulfonic acid, octocrylene (Parsol 340, DSM), oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, Eusolex.TM. 6300, avobenzone (Parsol 1789, DSM), avobenzone, PABA, octyldimethyl-PABA, Phenylbenzimidazole sulfonic acid, Cinoxate, Dioxybenzone (Benzophenone-8), Oxybenzone (Benzophenone-3), Homosalate, Menthyl anthranilate, Octisalate, Sulisobenzone, Trolamine salicylate, Terephthalylidene Dicamphor Sulfonic Acid, 4-Methylbenzylidene camphor, Methylene Bis-Benzotriazolyl Tetramethylbutylphenol, Bis-ethylhexyloxyphenol methoxyphenol triazine, bisimidazylate, Drometrizole Trisiloxane, Octyl triazone, Diethylamino Hydroxybenzoyl Hexyl Benzoate, Iscotrizinol, Polysilicone-15, Amiloxate, Ethylhexyl Dimethoxybenzylidene Dioxoimidazolidine Propionate, and mixtures thereof.

In addition to a UV-active, the non-volatile oil may comprise an ancillary oil which may be a solvent for one or more of the UV-active oils. The ancillary oil may provide desirable cosmetic properties such as emolliency and a good "skin feel." A preferred, but non-limiting ancillary oil is isopropyl myristate.

Non-volatile cosmetic emollient oils having a relatively high boiling point and function as a skin feel modifiers include, but are not hydrocarbons, fatty alcohols, fatty acids, non-volatile silicone oils, and esters such as glycerides and glycol esters.

Suitable ancillary oils include, but are not limited to isotridecyl isononanoate, isostearyl isostearate, isocetyl isosteatrate, isopropyl isostearate, isodecyl isonoanoate, cetyl octanoate, isononyl isononanoate, isocetyl myristate, isotridecyl myristate, isopropyl myristate, isostearyl palmitate, isocetyl palmitate, isodecyl palmitate, isopropyl palmitate, octyl palmitate, caprylic/capric acid triglyceride, glyceryl tri-2-ethylhexanoate, neopentyl glycol di(2-ethyl hexanoate), diisopropyl dimerate, tocopherol, tocopherol acetate, avocado oil, camellia oil, turtle oil, macadamia nut oil, corn oil, mink oil, olive oil, rapeseed oil, egg yolk oil, sesame oil, persic oil, wheat germ oil, pasanqua oil, castor oil, linseed oil, safflower oil, cotton seed oil, perillic oil, soybean oil, peanut oil, tea seed oil, kaya oil, rice bran oil, china paulownia oil, Japanese paulownia oil, jojoba oil, rice germ oil, glycerol trioctanate, glycerol triisopalmiatate, trimethylolpropane triisostearate, glycerol tri-2-ethylhexanoate, pentaerythritol tetra-2-ethylhexanoate, lanolin, liquid lanolin, liquid paraffin, squalane, vaseline, and mixtures thereof. Commercially available oils include, for example, tridecyl isononanoate with tradename Crodamol TN available from Croda, Hexalan available from Nisshin Seiyu, and tocopherol acetates available from Eisai.

Non-volatile cosmetic emollients may include waxes such as, but not limited to paraffin wax, microcrystalline wax, ozokerite wax, ceresin wax, carnauba wax, candelilla wax, and eicosanyl behenate.

Non-volatile silicon oils may be used including, but not limited to polymethylphenylsiloxane, polydiphenylsiloxane, polydiethylsiloxane, polydimethylsiloxane (dimethicone). For purposes of the present disclosure, a non-volatile silicon oil is defined as one that has a kinematic viscosity greater than 10 centiStokes (cSt).

Suitable ancillary oils include polyalkyl or polyaryl siloxanes as disclosed in U.S. 6,936,241, the disclosure of which is incorporated by reference herein in its entirety.

Suitable ancillary oils useful herein include the various grades of mineral oils. Mineral oils are liquid mixtures of hydrocarbons that are obtained from petroleum. Specific examples of suitable hydrocarbons include paraffin oil, mineral oil, dodecane, isododecane, hexadecane, isohexadecane, eicosene, isoeicosene, tridecane, tetradecane, polybutene, polyisobutene, and mixtures thereof.

The non-volatile oil may not comprise a "volatile" silicone oil. A specifically excluded volatile silicone oil is decamethylcyclopentanasilaxane, commonly known as "D5."

The foregoing description of the embodiments illustrates and describes the preferred embodiments but, as mentioned above, it is to be understood that the embodiments are capable of use in various other combinations, modifications, and environments and is capable of changes or modifications within the scope of the inventive concept as expressed herein, commensurate with the above teachings and/or the skill or knowledge of the relevant art. The embodiments described hereinabove are further intended to explain best modes known of practicing them and to enable others skilled in the art to utilize the embodiments in such, or other, embodiments and with the various modifications required by the particular applications or uses. Accordingly, the description is not intended to limit the embodiments to the form disclosed herein. Also, it is intended that the appended claims be construed to include alternative embodiments.

Throughout this application, various references including publications, patents, and pre-grant patent application publications are referred to. Disclosures of these publications in their entireties are hereby incorporated by reference into this application to more fully describe the state of the art to which the embodiments pertain. It is specifically not admitted that any such reference constitutes prior art against the present application or against any claims thereof. All publications, patents, and pre-grant patent application publications cited in this specification are herein incorporated by reference, and for any and all purposes, as if each individual publication or patent application were specifically and individually indicated to be incorporated by reference. In the case of inconsistencies the present disclosure will prevail.

The embodiments now will be explained in greater detail with reference to the following non-limiting examples.

### Examples

### Example 1 - Preparation of Surface Treated Powders

The present example discloses treatments of cellulose powder with various treatment agents.

Water was mixed together with the amount of dextrin palmitate listed in the table below, at about 50°C using a ROBOMIX^{®} disperser, commercially available from Primix Corporation, Osaka, Japan, for a period of about 15-30 minutes. To this homogenous mixture then were added commercially available cellulose powder, and mixed until homogenized. The temperature was increased to about 80°C, mixed about 3 times for 5 minutes each, and then held for about 6 hours. The samples then were tested for texture, and the texture was indicated as being Good or NG (not good). The results are shown in the Tables Below, using the following key:

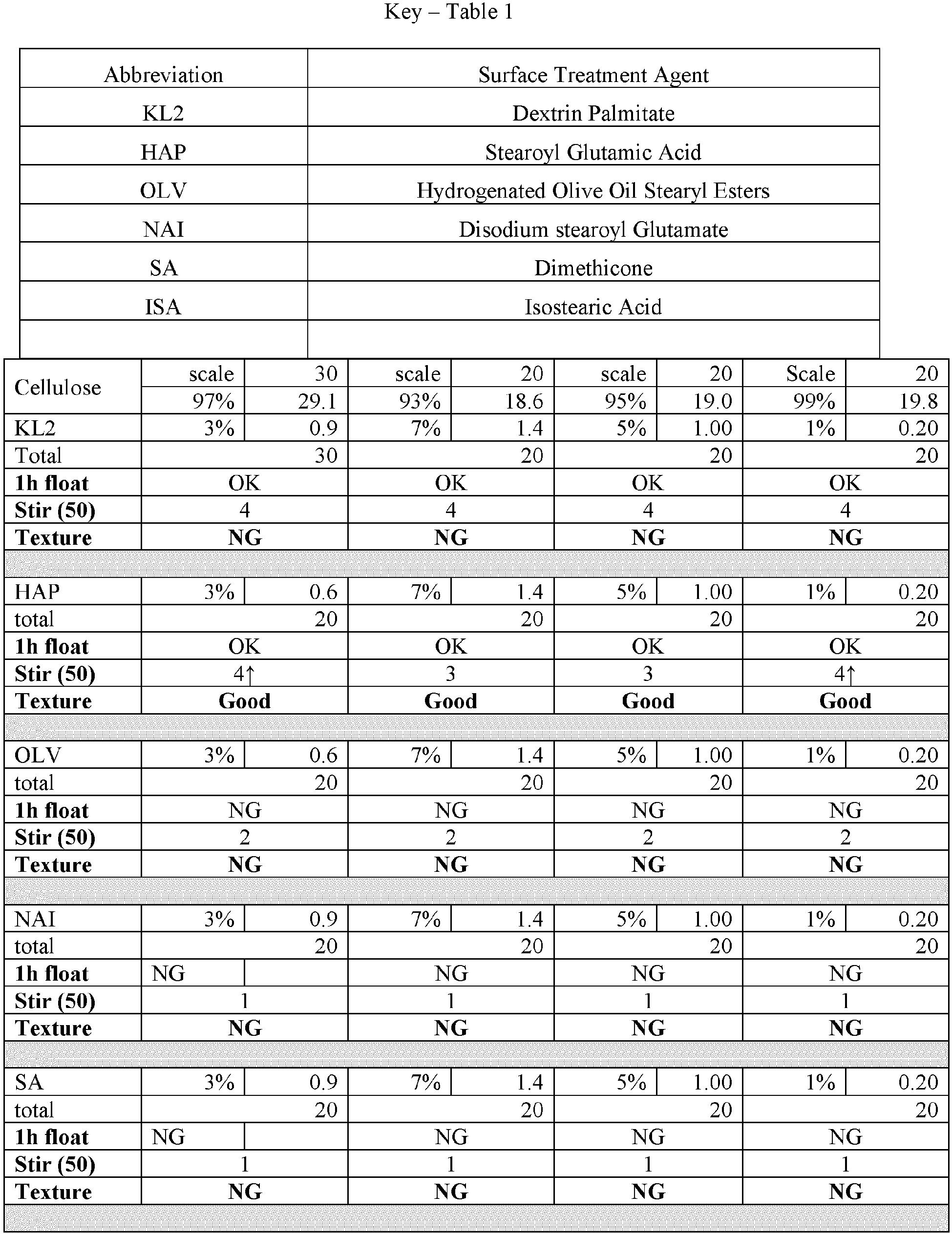

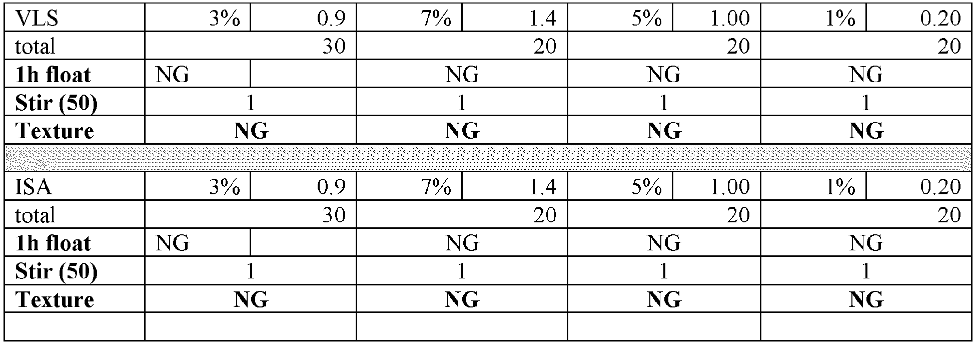

The data from the above table shows that treatment with a modified dextrin and a modified amino acid provides good float and good texture.

### Example 2 - Hydrophobicity of Surface Treated Powders

An oil was mixed together with the amount of dextrin palmitate listed in the table below, at about 50°C using a ROBOMIX^{®} disperser, commercially available from Primix Corporation, Osaka, Japan, for a period of about 15-30 minutes. To this homogenous mixture then were added commercially available cellulose powder, and mixed until homogenized. The temperature was increased to about 70°C, mixed about 4 times for 30 seconds each, and then allowed to stand for about 6 hours. The samples then were photographed to assess whether the powders were hydrophobic, which was indicated by the formation of a relatively clear liquid with white powder on the top, or were not, which was indicated by a milky or cloudy liquid with treated cellulose still dispersed.

The following table shows the amounts of cellulose and individual surface treatment agents.

**Table 2**

| | scale | 30 | scale | 20 | scale | 20 | scale | 20 | scale | 20 | scale | 20 | scale | 20 |
|---|---|---|---|---|---|---|---|---|---|---|---|---|---|---|
| Cellulose | 97% | 29.1 | 99.0% | 19.8 | 97.0% | 19.4 | 97.0% | 19.4 | 97.0% | 19.4 | 97.0% | 19.4 | 97.0% | 19.4 |
| KL2 (A) | 3% | 0.9 | | | | | | | | | | | | |
| HAP (B) | | | 1% | 0.2 | | | | | | | | | | |
| OLV (C) | | | | | 3% | 0.6 | | | | | | | | |
| NAI (D) | | | | | | | 3% | 0.6 | | | | | | |
| SA (E) | | | | | | | | | 3% | 0.6 | | | | |
| VLS (F) | | | | | | | | | | | 3% | 0.6 | | |
| ISA (G) | | | | | | | | | | | | | 3% | 0.6 |
| total | | 30 | | 20 | | 20 | | 20 | | 20 | | 20 | | 20 |
| **1h float** | OK | | OK | | NG | | NG | | NG | | NG | | NG | |
| **50xstir** | 4 | | 4↑ | | 2 | | 1 | | 1 | | 1 | | 1 | |

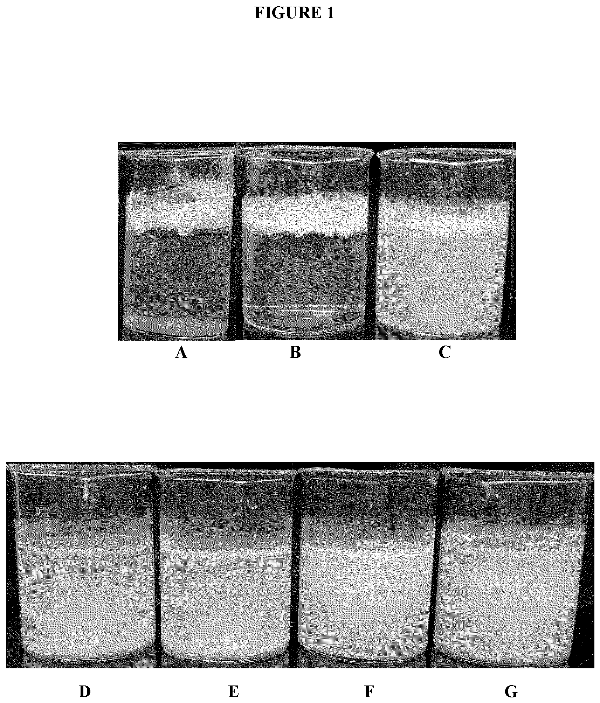

Photographs of the mixing vessel were taken to show whether the surface treated cellulose powder separated from the aqueous solution, thus indicating its hydrophobicity, or whether it remained dispersed in the solution, the results of which are shown in Figure 1 as Samples A-G. The product obtained then was separated from the mixture and dried at a temperature of about 105 °C for 16 hours to produce about 100 grams of cellulose powder treated with dextrin palmitate. As can be seen from Figure 1, only the cellulose powder treated with the modified dextrin or the modified amino acid (KLD - Sample A, or HAP - Sample B) possessed the requisite hydrophobicity.

The hydrophobic, surface treated cellulose powders of samples A and B then were dispersed in an emulsion, mixed, and then held at either room temperature, at 50°C, or -20°C for 4 weeks. Photographs then were taken for each emulsion, using 4 different samples of each at each temperature. The photographs depict the stability of the treated cellulose powder in an emulsion, in which the more stable emulsion will appear more uniform with small droplets dispersed therein, and less stable emulsions have larger droplets of dispersed material.

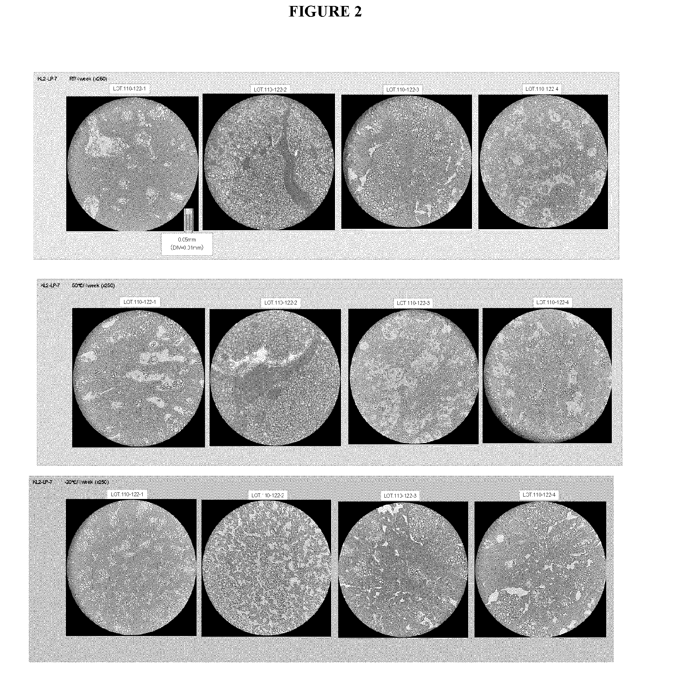

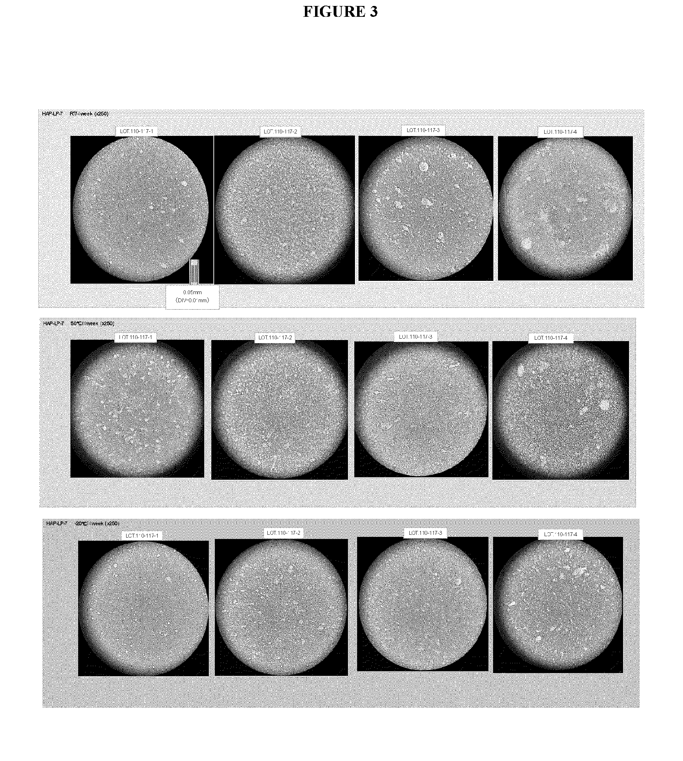

Figure 2 provides photographs of Sample A, cellulose treated with modified dextrin that was dispersed in an emulsion, at various testing conditions, and Figure 3 provides photographs of Sample B, cellulose treated with modified amino acid, hat was dispersed in an emulsion, at various testing conditions. It can be seen from comparing Figures 2 and 3 that the cellulose powders treated with modified amino acids depicted superior stability in an emulsion, when compared to cellulose powders treated with modified dextrin, but that both had good stability, when compared to other surface treatment agents (C-G). The cellulose powders treated with the other surface treatment agents, many of which are well-known and used in the art, were not suitable for dispersion in an emulsion.

The results of these experiments were unexpected insofar as well-known surface treatment agents known in the cosmetics industry for treating cosmetic powders such as substrates and pigments, such as dimethicone, isostearic acid, disodium stearoyl glutamate, were not effective in the rendering the cellulose powder particle surfaces hydrophobic.

The invention has been described with reference to particularly preferred embodiments. Those having ordinary skill in the art will readily appreciate that various modifications may be made to the invention without departing from the spirit and scope thereof.

## Claims

1. A hydrophobic cellulose powder comprising:
(a) from about 75% to about 99.5% by weight of cellulose powder particles; and
(b) from about 0.5% to about 25% by weight of a surface treatment agent selected from modified dextrin or a modified amino acid,
wherein the hydrophobic cellulose powder is relatively insoluble in an aqueous medium, and can be dispersed in an emulsion to provide a stable emulsion for 4 weeks or more.

2. The hydrophobic cellulose powder of claim 1, wherein the surface treatment agent is a modified dextrin.

3. The hydrophobic cellulose powder of claim 2, wherein the modified dextrin is a fatty acid ester of dextrin, wherein the fatty acid has from about 8 to about 20 Carbon atoms.

4. The hydrophobic cellulose powder of claim 3, wherein the modified dextrin is dextrin palmitate.

5. The hydrophobic cellulose powder of claim 1, wherein the surface treatment agent is a modified amino acid.

6. The hydrophobic cellulose powder of claim 5, wherein the modified amino acid is an acylamino acid in which a fatty acid is conjugated, or amidated to an amino acid.

7. The hydrophobic cellulose powder of claim 6, wherein the amino acid is selected from the group consisting of glutamic acid, sarcosine, aspartic acid, alanine, valine, leucine, isoleucine, proline, hydroxyproline, glycine, serine, cysteine, cystine, threonine, histidine and salts thereof.

8. The hydrophobic cellulose powder of claim 7, wherein the amino acid is selected from glutamic acid, sarcosine, aspartic acid, glycine, and lysine.

9. The hydrophobic cellulose powder of claim 8, wherein the amino acid is glutamic acid.

10. The hydrophobic cellulose powder of claim 6, wherein the fatty acid is a C₈-C₂₀ fatty acid.

11. The hydrophobic cellulose powder of claim 10, wherein the fatty acid is a C₁₂-C₁₈ fatty acid.

12. The hydrophobic cellulose powder of claim 1, wherein the modified amino acid is selected from the group consisting of stearoyl amino acid, or stearoyl glutamic acid, stearoyl aspartic acid, stearoyl lysine, stearoyl glycine, and mixtures thereof.

13. The hydrophobic cellulose powder of claim 12, wherein the modified amino acid is stearoyl glutamic acid.

14. A method of making a hydrophobic cellulose powder comprising:
(a) preparing a mixture of from about 0.5% to about 25% by weight of a surface treatment agent selected from modified dextrin or a modified amino acid, and from about 75% to about 99.5% by weight by weight of the hydrophobic cellulose powder in an agent suitable to dissolve or disperse the surface treatment agent, wherein the weight percentages are based on the total weight of the surface treatment agent and cellulose powder;
(b) mixing the mixture of (a) with agitation to uniformly disperse the powder in the mixture and form a uniformly dispersed surface treated cellulose powder mixture;
(c) heating the uniformly dispersed surface treated cellulose powder mixture either during (b) or after (b); and
(c) separating the hydrophobic cellulose powder from the uniformly dispersed surface treated cellulose powder mixture, and optionally drying to form a hydrophobic cellulose powder.

15. A cosmetic composition comprising:
(a) at least one hydrophobic cellulose powder comprising: (i) from about 75% to about 99.5% by weight of cellulose powder particles; and (ii) from about 0.5% to about 25% by weight of a surface treatment agent selected from modified dextrin or a modified amino acid; and
(b) a cosmetically acceptable carrier.

16. The cosmetic composition of claim 14, wherein the composition is in the form selected from the group consisting of a powder foundation, liquid foundation, point makeup, lip, mascara, eyeliner, skin care products which are skin cream, hair care products which are shampoo, conditioner, treatment and hair styling products, hair color products, cleansing products which are a body soap, hand soap and facial cleanser.
